# EUROPEAN PATENT APPLICATION

(11) **EP 2 439 523 A2**
(43) Date of publication of application: **11.04.2012**
(21) Application number: 10783522.5
(22) Date of filing: 30.04.2010
(51) Int. Cl.: G01N 27/26, G01N 27/416, G01N 33/48

(54) **BIOSENSOR FOR MEASURING BIOMATERIAL**

(30) Priority: 02.06.2009 KR 20090048676
(71) Applicant: Ceragem Medisys Inc., Gyeonggi-do 435-776 (KR)
(72) Inventor: LEE, Jin-Woo, Suwon-si Gyeonggi-do 440-330 (KR); CHOI, Jae-Kyu, Daejeon 302-280 (KR)
(74) Representative: Stöckeler, Ferdinand
(86) International application number: PCT/KR2010/002738
(87) International publication number: WO 2010/140773

(57) **Abstract**

The present invention relates to an apparatus for measuring a biomaterial, comprising: a first substrate having a recess in one side thereof; a second substrate having reference and operating electrodes where a biochemical reaction of a biomaterial occurs, and first and second delivery electrodes delivering electric signals from the reaction to a detector; and reaction reagents fixed at the second substrate causing the reaction with the biomaterial, wherein the first or the second substrate has a tilted surface toward a sample-inlet in a height direction. The second substrate is attached to the first substrate such that a portion of the recess forms the sample-inlet, and the reference and operating electrodes are directed toward the recess. The tilted surface prevents the sample-inlet from being blocked by a finger of the user when introducing the biomaterial through the sample-inlet in the event the used substrate has three-dimensional structures with substantial thickness.

## Description

### [Technical Field]

The present invention relates to an apparatus for measuring a biomaterial, and more particularly, to an apparatus for measuring a biomaterial, which prevents a sample inlet from being blocked by the contact of a finger when a substrate constituting a biosensor has a three-dimensional structure with a substantial thickness.

### [Background Art]

Biosensors are measuring instruments that examine the properties of a substance using functions of an organism. These biosensors are excellent in sensitivity and reaction specificity because the biosensors use a biomaterial as detecting element. Thus, the biosensors are broadly used in various fields such as clinical chemical analysis, process instrumentation of bioindustry, environment instrumentation, stability evaluation of chemicals, and so on, and their usage is continuing to spread. Particularly, a variety of biosensors are used in a medical diagnostic field to analyze samples, particularly bio-samples. The biosensors are divided into enzyme assay biosensors and immunoassay biosensors according to the kind of detecting element, and into optical biosensors and electrochemical biosensors according to a method of quantitatively analyzing a target substance within a bio-sample.

The enzyme assay biosensors are designed to use a specific reaction between an enzyme and a substrate and a specific reaction between an enzyme and an enzyme inhibitor, and the immunoassay biosensors are designed to use a specific reaction between an antigen and an antibody.

The optical biosensors are widely used to measure a concentration of a target material by measuring transmittance, absorbance, or alteration in wavelength. The optical biosensors have an advantage in that, since reaction mechanisms of various materials to be analyzed have already been known and measurement is made after a reaction takes place for a sufficient time, a deviation in measurement time is low. In contrast, the optical biosensors have a disadvantage in that they require a longer measurement time and a greater quantity of samples than the electrochemical biosensors. Further, the optical biosensors have other disadvantages in that measured results are influenced by turbidity of a sample, and it is difficult to miniaturize an optical unit.

The electrochemical biosensors are used to measure a concentration of a target material by measuring an electric signal obtained from a reaction. The electrochemical biosensors have advantages in that it is possible to amplify a signal using a very small quantity of sample, they are easy to miniaturize, it is possible to stably obtain a measured signal, and they can be easily combined with a telecommunication instrument. However, the electrochemical biosensors have disadvantages in that an electrode manufacturing process is additionally required, the cost of production is high, and a measured signal is very sensitive to response time.

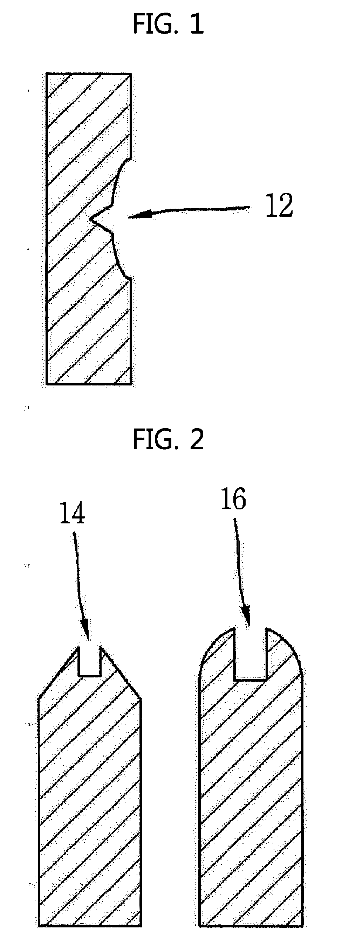

Meanwhile, in the case of existing disposal biosensors having a capillary for introducing a sample, a sample inlet has a flat cross section. For this reason, when the sample inlet is touched with, for example, a finger in order to introduce blood, the sample inlet is completely blocked, and thus the sample is no longer introduced. Accordingly, Roche manufactures a biosensor in which an indentation and a notch are formed so as to prevent a sample inlet from being blocked by a finger, as shown in FIG.1. Further, Arkray manufactures a biosensor in which a sample inlet 14 or 16 is formed in a pointed shape so as to prevent the sample inlet 14 or 16 from being blocked by a finger, as shown in FIG. 2.

Since all of these existing biosensors have a two-dimensional structure using a thin film, only two-dimensional processing is possible to realize a notch in a plane. However, when a substrate used to manufacture the biosensor has a three-dimensional structure with a substantial thickness, it is insufficient to merely form such a notch in the sample inlet in order to prevent the sample inlet from being blocked by a finger.

Further, the existing biosensors have a problem that, when the finger is put to the sample inlet and then is released before the capillary is not sufficiently filled with blood, a capillary gap is sufficiently filled with the sample, thereby providing inconsistent measurement results.

### [Disclosure]

### [Technical Problem]

The present invention is directed toward preventing a sample inlet from being blocked when a sample is introduced into the sample inlet if a substrate used has a three-dimensional structure with a substantial thickness.

The present invention is directed toward overcoming a problem that has an influence on measurement results because a capillary gap is not sufficiently filled with a sample when a finger is released from a sample inlet before the capillary gap is sufficiently filled with the sample.

The objectives of the present invention are not limited to those mentioned above. Other objectives and advantages of the present invention which are not disclosed will be understood from the following description, and be apparent with reference to the embodiments of the present invention. Also, it is obvious to those skilled in the art that the objectives and advantages of the present invention will be realized by the means as claimed and combinations thereof

### [Technical Solution]

In order to achieve the above objectives, according to one aspect of the present invention, there is provided an apparatus for measuring a biomaterial, which includes: a first substrate having a recess formed in one side surface thereof; a second substrate having a plurality of reaction electrodes in which a biochemical reaction of a sample occurs and a plurality of delivery electrodes transmitting a signal generated by the biochemical reaction to a detector; and a reaction reagent located in the recess and causing the biochemical reaction with the sample, wherein the second substrate is attached to the first substrate such that the reaction electrodes are directed toward the recess and the recess forms at least one vent slit in combination with at least one edge surface of the second substrate, the first and second substrates are attached to form a sample inlet and a reaction chamber, and the first or second substrate has a surface tilted toward the sample inlet in a height direction.

According to another aspect of the present invention, there is provided an apparatus for measuring a biomaterial, which includes: a first substrate; a second substrate attached to the first substrate so as to form a sample inlet and a reaction chamber; a plurality of electrodes located in the reaction chamber and attached to the first or second substrate; and a reaction reagent located in the reaction chamber and causing the biochemical reaction with the sample, wherein the first or second substrate has a surface tilted toward the sample inlet in a height direction.

### [Advantageous Effects]

According to the present invention, a surface tilted toward a sample inlet in a height direction can prevent the sample inlet from being blocked by a finger when a sample is introduced into the sample inlet of a biosensor in which a substrate used has a three-dimensional structure with a substantial thickness. Further, the tilted surface additionally retains the sample, so that a capillary gap can be sufficiently filled with the sample even when a finger is released before the capillary gap is sufficiently filled with the sample, and thus constant measured results can be obtained from the same sample.

### [Description of Drawings]

FIG. 1 is a view for explaining a conventional biosensor having an indentation and a notch.
FIG. 2 is a view for explaining a conventional biosensor having a protruding sample inlet.
FIG. 3 is a view for explaining the structure of a biosensor according to the present invention.
FIG. 4 is a view for explaining embodiments of the present invention, each of which includes a surface tilted toward a sample inlet in a height direction.
FIG. 5 is a view for explaining other embodiments of the present invention in which a round protrusion or recess is formed on a tilted surface.
FIG. 6 is a view for explaining still other embodiments of the present invention in which one or more round protrusions or recesses are formed on a tilted surface.
FIG. 7 is a view for explaining yet other embodiments of the present invention in which one or more rod-shaped protrusions or recesses are formed on a tilted surface.
FIG. 8 is a view for explaining yet other embodiments of the present invention in which a protrusion or notch is formed on an edge of a tilted surface.
FIG. 9 is a view for explaining yet other embodiments of the present invention in which a notch is formed on an edge of a tilted surface and in which one or more round or rod-shaped protrusions or recesses are formed on the tilted surface.
FIG. 10 is a view for explaining another embodiment in which reaction electrodes and delivery electrodes of the present invention are electrically connected to each other.

### [Best Mode]

### [Mode for Invention]

The foregoing and other objects, features and advantages of the invention will become more apparent from the following detailed description when read in conjunction with the accompanying drawings. Accordingly, it will be easily understood by those skilled in the art that the invention can be modified in various forms without departing from the technical spirit of the invention. In the following description, well-known functions or constructions are not described in detail since they would obscure the invention in unnecessary detail. Exemplary embodiments of the invention will be described below in detail with reference to the accompanying drawings.

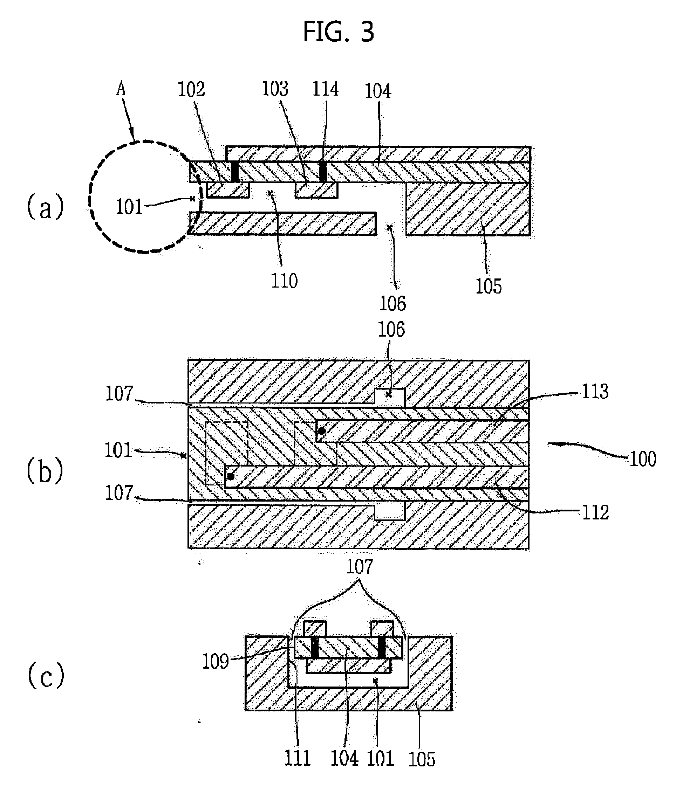

FIG. 3 is a view for explaining the structure of a biosensor according to one embodiment of the present invention. FIG. 3(a) is a side view of the biosensor, FIG. 3(b) is a plan view of the biosensor, and FIG. 3(c) is a front view of the biosensor.

Referring to FIG. 3, the biosensor according to one embodiment of the present invention includes a first substrate having a recess and a sample inlet, and a second substrate having a plurality of reaction electrodes and a plurality of delivery electrodes.

The first substrate 105 is a bonding substrate that serves as a physical support, and is provided with a recess 110 in one side surface thereof. A portion, preferably one end, of the recess 110 forms a sample inlet. The second substrate 104 is a reaction substrate having a reference electrode 102, a working electrode 103, a first delivery electrode 112, and a second delivery electrode 113. A reaction reagent (not shown) is immobilized to the second substrate 104 across the reference electrode 102 and the working electrode 103, so that it is located in the recess 110. A biochemical reaction between the reaction reagent and the sample takes place around the reference and working electrodes 102 and 103, on which the reaction reagent is immobilized. The first delivery electrode 112 is electrically connected to the reference electrode 102, and the second delivery electrode 113 is electrically connected to the working electrode 103. Thereby, an electric signal generated from the reference and working electrodes 102 and 103 by the biochemical reaction between the reaction reagent and the sample is transmitted to a detector. Herein, the electrodes, such as the reference electrode and the working electrode, which participate in the biochemical reaction are generically referred to as "reaction electrodes," which are distinguished from the delivery electrodes that transmit the electric signal generated by the biochemical reaction to a measuring apparatus. The reference electrode is generally called a "counter electrode" in the related art.

Referring to FIG. 3(a) to 3(c), the first substrate 105 having the recess 110 whose front side is opened and the second substrate 104 having the electrodes and a planar structure are bonded to each other, so that the front side of the recess is formed as the sample inlet 101. Alternatively, in the first substrate having the recess whose front side is closed, only a portion of the recess may be covered by the second substrate, and the other parts of the recess may be open to form the sample inlet.

When the first and second substrates 105 and 104 are bonded to each other, a reaction chamber is formed in a capillary structure. That is, the first substrate 105 is covered by the second substrate 104, so that the recess 110 formed in the first substrate is formed as a channel or a reaction chamber having a capillary structure. The second substrate 104 is attached to the first substrate 105 such that the reference electrode 102 and the working electrode 103 are directed toward the recess 110 and that at least one vent slit 107 is formed by a combination of the recess 110 and at least one edge surface 109 of the second substrate 104. The vent slit 107 continuously extends from the sample inlet 101 in a lengthwise direction of the biosensor 100. Herein, the lengthwise direction of the biosensor 100 refers to a direction in which the sample is introduced into the recess 110 or the reaction chamber. The lengthwise direction of the biosensor 100 is equivalent to a lengthwise direction of the sample inlet 101, so that the two directions are compatible with each other herein.

The reference electrode 102 and the working electrode 103 are formed on a surface of the second substrate 104 which is directed toward the recess 110, and the first and second delivery electrodes 112 and 113 are formed on the opposite surface of the second substrate 104. The first and second delivery electrodes 112 and 113 are electrically connected to the reference electrode 102 and the working electrode 103 via conductors 114 passing through the second substrate 104, respectively.

In this embodiment, the first and second delivery electrodes 112 and 113 and the reference electrode 102 and the working electrode 103 are formed on the respective different surfaces of the second substrate 104, but they may be formed on the same surface of the second substrate. Further, in this embodiment, in the recess 110 formed in the first substrate 105, a vent hole 106 may be formed in the other end of the front side of the recess on which the sample inlet is formed. Alternatively, the vent hole 106 may be formed in the second substrate 104 rather than the first substrate 105, or may not be formed in any substrate.

The sample is introduced into the reaction chamber by a capillary phenomenon. The capillary phenomenon occurs between a surface of the second substrate 104 which is directed toward the recess 110 and a bottom surface of the recess 110 of the first substrate 105 as well as between the edge surface 109 of the second substrate 104 and a wall 111 of the recess 110. In detail, the one or more vent slits 107 formed between the edge surface 109 of the second substrate 104 and the wall 111 of the recess 110 serve as an air outlet when the sample is introduced. Thus, the vent slit 107 discharges air in the reaction chamber toward the outside and simultaneously introduces the sample into the reaction chamber by means of the capillary phenomenon, thereby making it faster to introduce the sample into the reaction chamber.

The second substrate 104 is physically isolated within a gap where the sample reacts with the reaction reagent. That is, unlike existing biosensors, the second substrate 104 is not in contact with the other substrate due to the vent slit 107. In this embodiment, the vent slit 107 is not formed by separately processing a specific substrate, but is three-dimensionally formed as a result of adjusting a positional relationship between the second substrate 104 and the first substrate 105. A gap of the vent slit 107 is easily adjusted by a thickness of the second substrate 104.

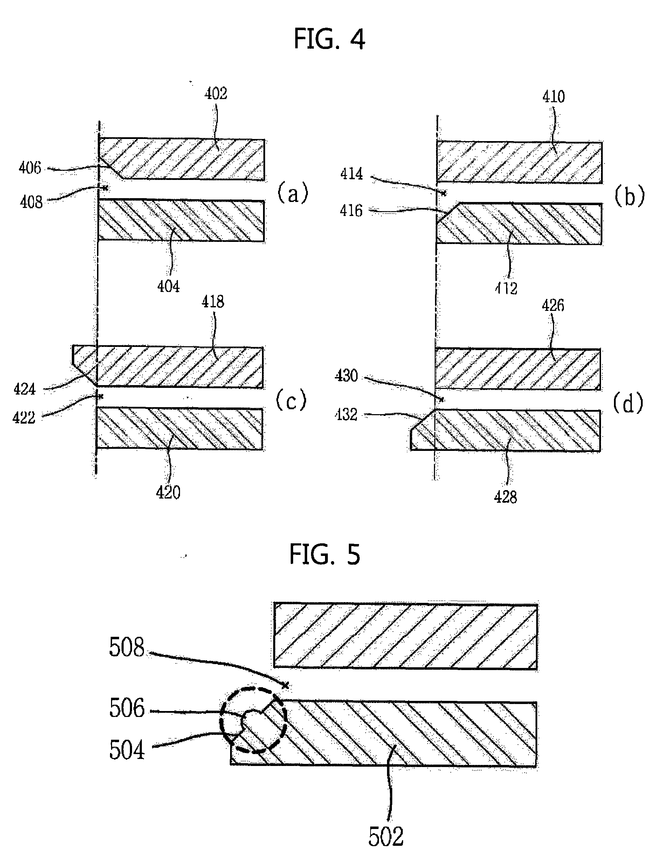

FIG. 4 is a view for explaining embodiments of the present invention, each of which includes a surface tilted toward a sample inlet in a height direction, and is an enlarged view of a dashed circle A of FIG. 3.

FIG. 4(a) shows a biosensor in which a surface 408 tilted toward a sample inlet 406 in a height direction is formed on a second substrate 402. The sample inlet 406 is formed by the second substrate 402 and a first substrate 404. The second substrate 402 and the first substrate 404 are aligned with each other at ends thereof. FIG. 4(b) shows a biosensor in which a surface 416 tilted toward a sample inlet 414 in a height direction is formed on a first substrate 412. The sample inlet 414 is formed by a second substrate 410 and the first substrate 412. The second substrate 410 and the first substrate 412 are aligned with each other at ends thereof.

FIG. 4(c) shows a biosensor in which a surface 424 tilted toward a sample inlet 422 in a height direction is formed on a second substrate 418. The sample inlet 422 is formed by the second substrate 418 and a first substrate 420. An end of the second substrate 418 having the tilted surface 424 protrudes forward farther than an end of the first substrate 420. FIG. 4(d) shows a biosensor in which a surface 432 tilted toward a sample inlet 430 in a height direction is formed on a first substrate 428. The sample inlet 430 is formed by a second substrate 426 and a first substrate 428. An end of the first substrate 428 having the tilted surface 432 protrudes forward farther than an end of the second substrate 426. As shown in FIG. 4(c) or 4(d), when the ends of the second and first substrates are not aligned with each other, this provides a great effect of preventing the sample inlet from being blocked by a finger, but has a disadvantage in that an area of the electrode is reduced because the electrode is formed on the short substrate.

As shown in FIG. 4, when the surface tilted toward the sample inlet in a height direction is formed on the first or second substrate, the sample inlet is not blocked by a finger when the finger is put to the sample inlet in order to introduce the sample, so that it is easy to introduce the sample. Further, the tilted surface serves to additionally retain the sample like a reservoir, thereby causing the capillary to be sufficiently filled. As a result, measurement results of the samples become consistent.

FIG. 5 is a view for explaining another embodiment of the present invention in which a round protrusion is formed on a tilted surface. A tilted surface 504 is formed on an end of a first substrate 502, and a round protrusion 506 is formed in the middle of the tilted surface 504. The protrusion 506 formed on the tilted surface 504 is allowed to more effectively prevent a sample inlet 508 from being blocked by a finger and to additionally retain a sample by catching the sample on the protrusion 506. In FIG. 5, the protrusion is formed. Alternatively, a recess may be formed.

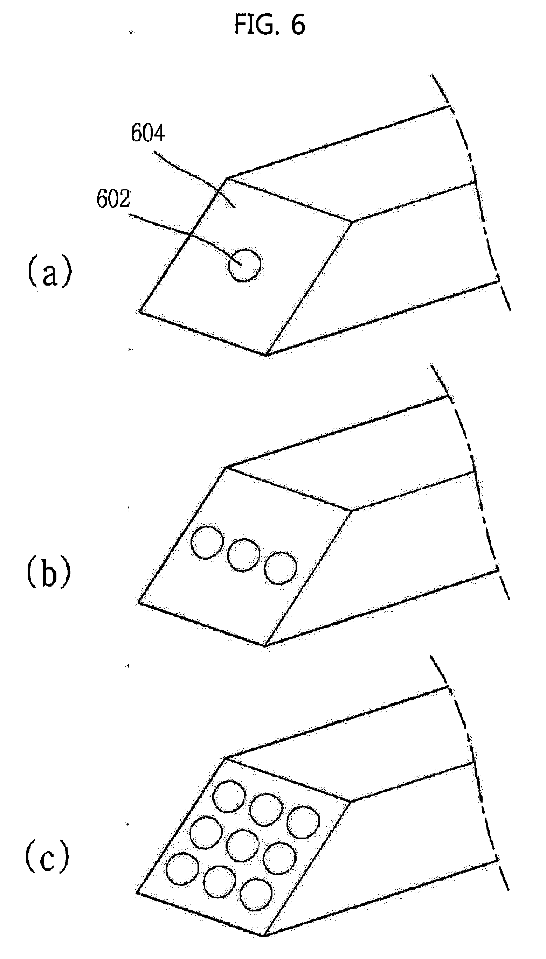

FIG. 6 is a view for explaining other embodiments of the present invention in which at least one protrusion is formed on a tilted surface. In FIG. 6(a), one round protrusion 602 is formed on a tilted surface 604. In FIG. 6(b), a plurality of round protrusions are formed on a tilted surface in a row. In FIG. 6(c), a plurality of round protrusions are formed on a tilted surface in an array pattern. The more round protrusions are formed on the tilted surface, the further a function of preventing a sample inlet from being blocked by a finger and a function of retaining a sample are improved. In FIG. 6, the protrusion is formed. Alternatively, a recess may be formed.

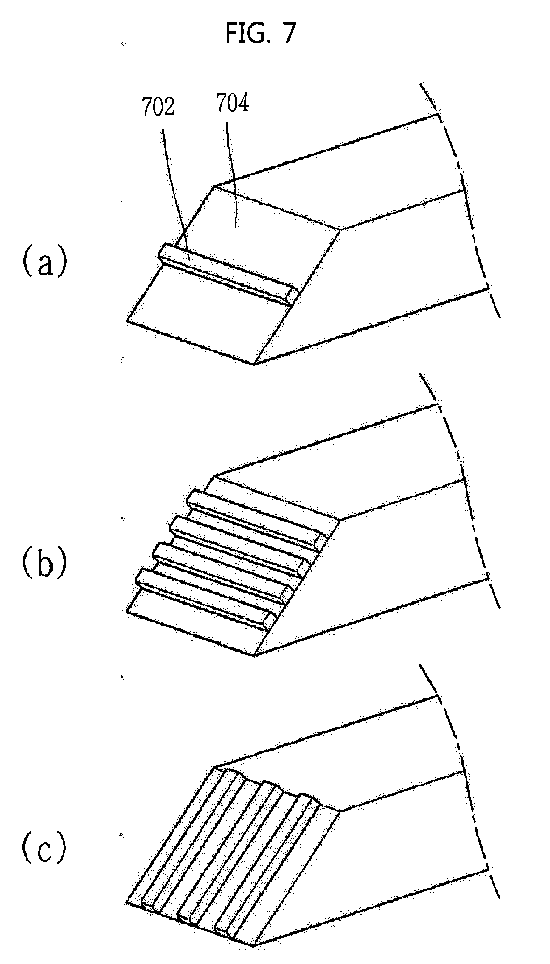

FIG. 7 is a view for explaining still other embodiments of the present invention in which at least one rod-shaped protrusion is formed on a tilted surface. In FIG. 7(a), one rod-shaped protrusion 702 is formed on a tilted surface 704. In FIG. 7(b), a plurality of rod-shaped protrusions are formed on a tilted surface in a horizontal direction. In FIG. 7(c), a plurality of rod-shaped protrusions are formed on a tilted surface in a vertical direction, i.e. in a height direction where they are directed toward a sample inlet. The more rod-shaped protrusions are formed on the tilted surface, the further a function of preventing the sample inlet from being blocked by a finger and a function of retaining a sample are improved. In FIG. 7, the protrusion is formed. Alternatively, a recess may be formed. Further, the tilted surface may be formed in a stepped shape.

Meanwhile, when the plurality of rod-shaped protrusions are formed on the tilted surface in a horizontal direction as in FIG. 7(b), the function of retaining the sample is improved. When the plurality of rod-shaped protrusions are formed on the tilted surface in a vertical direction as in FIG. 7(c), i.e. in a height direction where they are directed toward the sample inlet, a function of introducing the sample into a capillary gap is improved because the rod-shaped protrusions serve to guide the introduction of the sample.

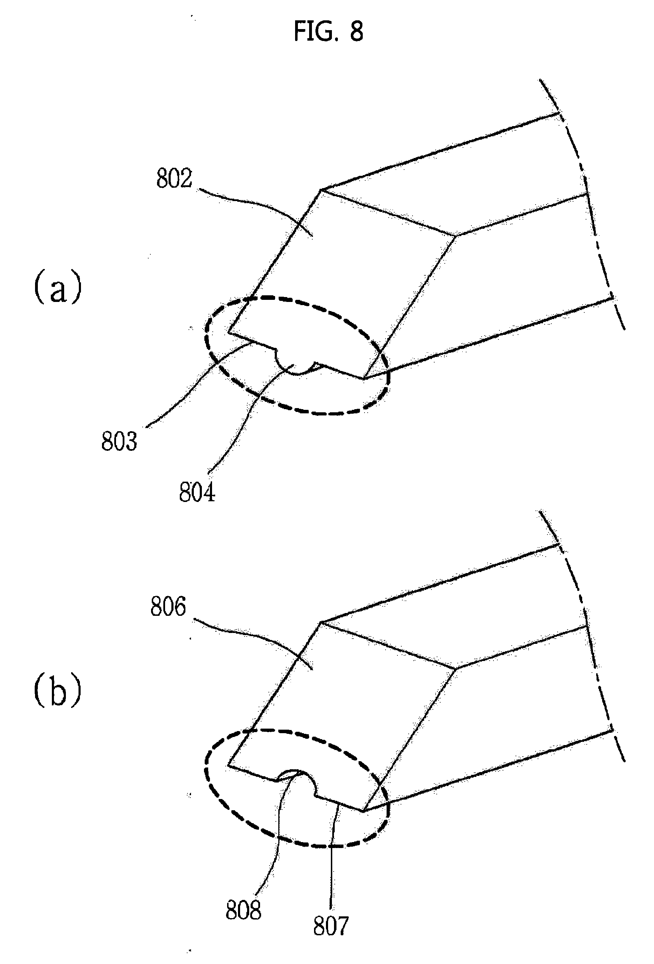

FIG. 8 is a view for explaining yet other embodiments of the present invention in which a protrusion or notch is formed on the edge of a tilted surface. In FIG. 8(a), a protrusion 804 is formed on a lower edge 803 of a tilted surface 802. In FIG. 8(b), a notch 808 is formed on a lower edge 807 of a tilted surface 806. The protrusion 804 and the notch 808 prevent a sample inlet from being blocked by a finger in cooperation with the tilted surfaces 802 and 806.

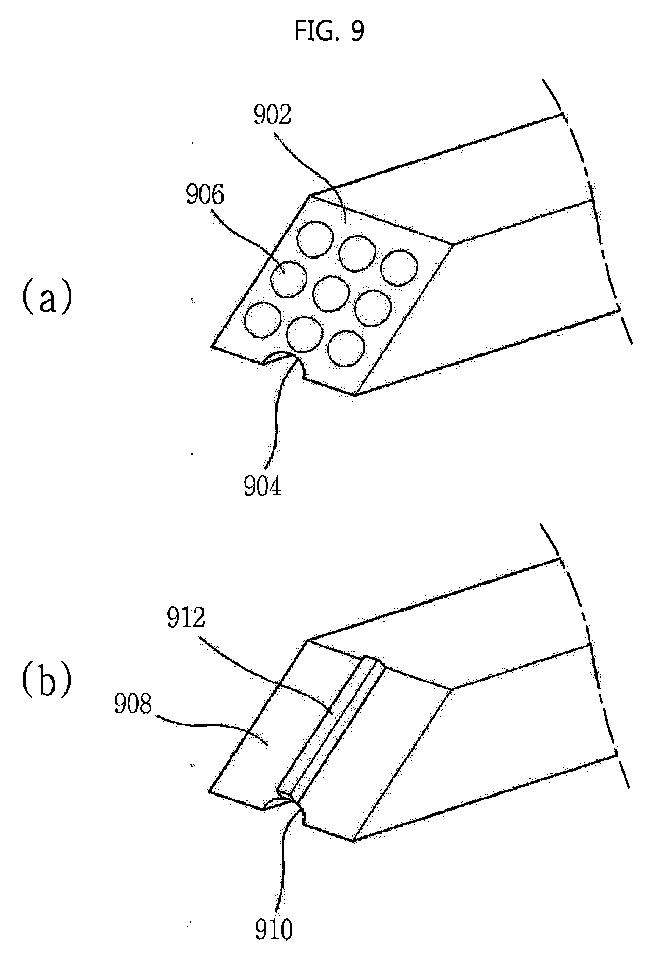

FIG. 9 is a view for explaining yet other embodiments of the present invention in which a notch formed on an edge of a tilted surface is combined with a round or rod-shaped protrusion formed in the tilted surface. In FIG. 9(a), a notch 904 formed on a lower edge of a tilted surface 902 is combined with round protrusions formed on the tilted surface 902 in an array pattern. In FIG. 9(b), a notch 910 formed on a lower edge of a tilted surface 908 is combined with a rod-shaped protrusion formed on the tilted surface 908 in a vertical direction (a height direction where the rod-shaped protrusion is directed toward the sample inlet). In FIG. 9, the round or rod-shaped protrusion is formed. Alternatively, a recess may be formed.

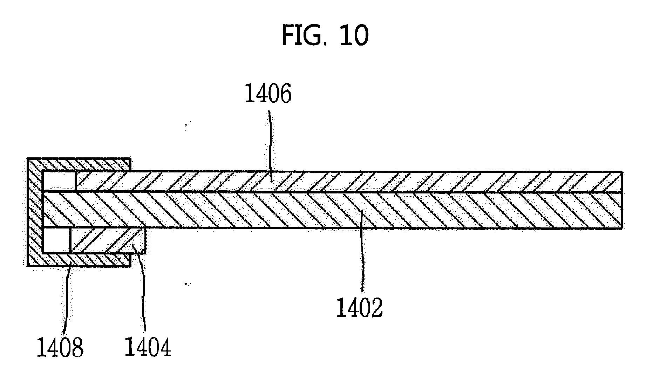

FIG. 10 is a view for explaining another embodiment in which reaction electrodes and delivery electrodes of the present invention are electrically connected to each other. As shown, a first or second delivery electrode 1406 formed on one side of a second substrate 1402 may be electrically connected to a reaction electrode 1404 via a conductive clamping member 1408 formed on an edge of the second substrate 1402. To stably fix the delivery electrode 1406 and the reaction electrode 1404, the conductive clamping member 1408 may be formed of a resilient material.

Although specific embodiments of the invention have been described with reference to the drawings, the invention is not limited to these specific embodiments. It is apparent to those skilled in the art that various modifications, additions and substitutions are possible without departing from the scope of the present invention which is intended to be defined by the appended claims. Particularly, the above embodiments are based on a two-electrode system. However, it will be understood that the present invention may be applied to all types of electrode systems including a three-electrode system.

## Claims

1. An apparatus for measuring a biomaterial, comprising:
a first substrate having a recess formed in one side surface thereof;
a second substrate having a plurality of reaction electrodes in which a biochemical reaction of a sample occurs and a plurality of delivery electrodes transmitting a signal generated by the biochemical reaction to a detector; and
a reaction reagent located in the recess and causing the biochemical reaction with the sample,
wherein the second substrate is attached to the first substrate such that the reaction electrodes are directed toward the recess and the recess forms at least one vent slit in combination with at least one edge surface of the second substrate,
the first and second substrates are attached to form a sample inlet and a reaction chamber, and
the first or second substrate has a tilted surface toward the sample inlet in a height direction.

2. An apparatus for measuring a biomaterial, comprising:
a first substrate;
a second substrate attached to the first substrate so as to form a sample inlet and a reaction chamber;
a plurality of electrodes located in the reaction chamber and attached to the first or second substrate; and
a reaction reagent located in the reaction chamber and causing the biochemical reaction with a sample,
wherein the first or second substrate has a tilted surface toward the sample inlet in a height direction.

3. The apparatus according to claim 1, wherein the recess is partially open.

4. The apparatus according to claim 1, wherein the recess forms at least one vent slit in combination with at least one edge surface of the second substrate.

5. The apparatus according to claim 1 or 2, wherein the tilted surface further includes at least one protrusion or recess.

6. The apparatus according to claim 5, wherein the protrusion or recess has a rod shape.

7. The apparatus according to claim 5, wherein the protrusion or recess is plural in number.

8. The apparatus according to claim 1 or 2, wherein the tilted surface has a stepped shape.

9. The apparatus according to claim 1 or 2, wherein the first or second substrate further includes a notch.

10. The apparatus according to claim 9, wherein the tilted surface further includes a rod-shaped protrusion or recess in a vertical direction, and the rod-shaped protrusion or recess is connected to the notch.
